# EUROPEAN PATENT SPECIFICATION

(11) **EP 4 603 064 B1**
(45) Date of publication and mention of the grant of the patent: **21.10.2026**
(21) Application number: 25157720.1
(22) Date of filing: 13.02.2025
(51) Int. Cl.: A61F 2/38, A61F 2/30

(54) **MODULAR ARTIFICIAL KNEE SYSTEM**
MODULARES KÜNSTLICHES KNIESYSTEM
SYSTÈME DE GENOU ARTIFICIEL MODULAIRE

(30) Priority: 13.02.2024 US 202418440041
(43) Date of publication of application: 20.08.2025
(73) Proprietor: Garino, Jonathan P., Villanova, PA 19085 (US)
(72) Inventor: Garino, Jonathan P., Villanova, PA 19085 (US)
(74) Representative: Scheele, Friedrich

(56) References cited:
- EP-A1- 0 103 697
- CN-A- 109 528 362
- US-A1- 2020 281 732
- US-A1- 2022 354 657

## Description

### FIELD OF THE INVENTION

This invention relates generally to artificial knee prostheses used for Total Knee Replacement (TKR), and more particularly, to a knee joint prosthesis having an artificial anterior cruciate ligament (ACL) and/or posterior cruciate ligament (PCL).

### BACKGROUND OF THE INVENTION

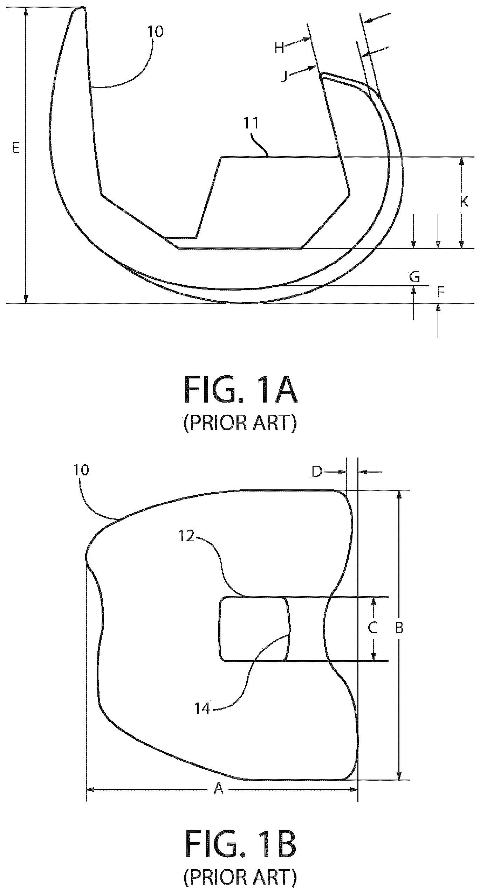

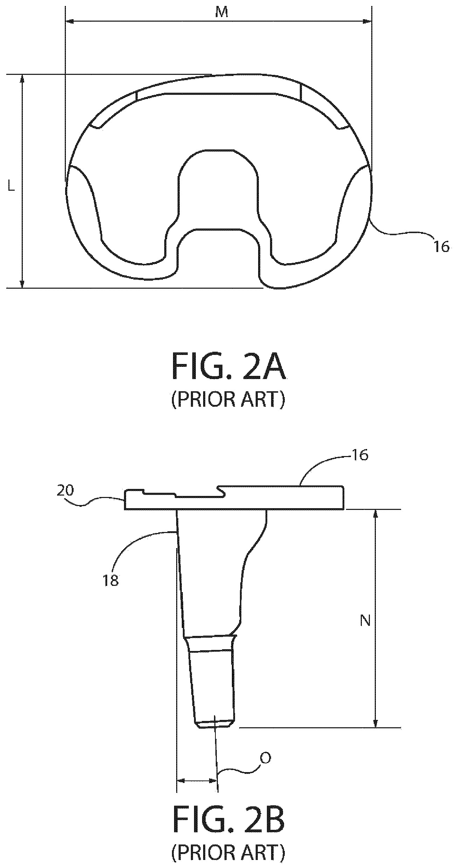

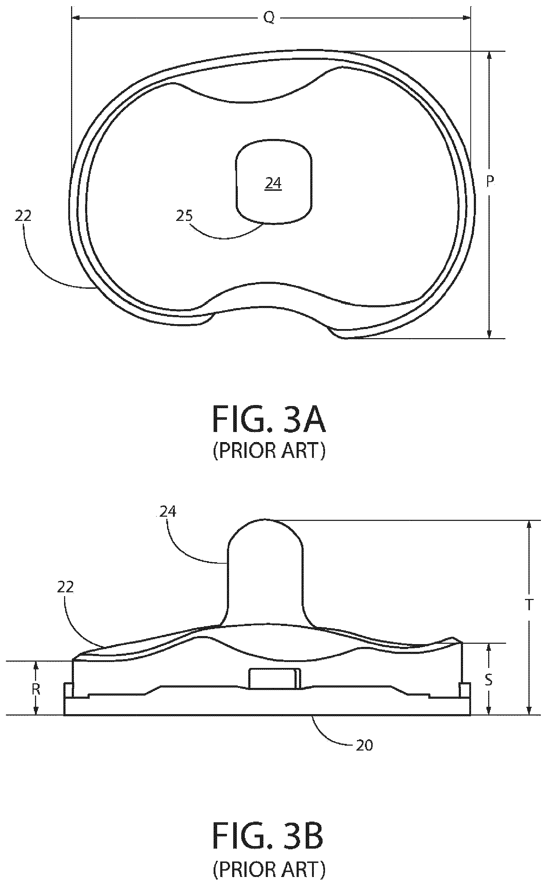

As is described in U.S. Patent App. Pub. No. 2017/0252173 to Garino, prosthetic knees generally include three main components, a femoral component (FIGs. 1A and 1B), which is attached to the distal end of the femur, a tibial baseplate (FIGs. 2A and 2B), which is implanted onto the proximal end of the tibia, and an articular tibial insert (FIGs. 3A and 3B), which is mounted onto the tibial baseplate and provides a frictional surface for the femoral component. The components are designed to simulate a joint and the associated mechanics of a human knee throughout the knee's range of motion. The components are generally provided in a variety of shapes with varying dimensions (identified as dimensions A-H and J-T in FIGs. 1A to 3B), so that a physician is able to select the optimal combination of components depending on the specific anatomy of the patient. The size and shape of the knee is dependent on various factors including age, gender, and size of the patient. Therefore, a fairly large inventory of components are generally made available, so that the prosthetic knee may be tailored for the patient.

During the course of a routine knee construction with a TKR, the ACL is removed in a vast majority of all cases and depending on the selected TKR design, the patient's PCL is either retained or substituted with some mechanism to replace the lost function of the PCL. Even when the PCL is retained, often a portion of the PCL must be cut or partially cut during surgery to aid in the balancing of the knee replacement. When the PCL is completely removed, the PCL is substituted by a post and cam mechanism.

A TKR generally comprises a femoral component 10, a tibial baseplate 16 having a post 18 that is implanted within a bore formed in the tibia, and an articular insert 22 that resides on a top mounting portion 20 of the tibial baseplate 16 for interfacing with the femoral component 10. Articular insert 22 may be either separate from tibial baseplate 16, as shown, or integrated with tibial baseplate 16 into a single component. The articular insert 22 and tibial baseplate 16 may be referred to herein either together or individually as a "tibial component."

Referring to FIGs. 1A, 1B, 3A, and 3B, an illustration of a typical design of a post and cam mechanism is provided. An articular insert 22 includes an extension 24 that protrudes into an opening 12 of the femoral component 10. A box 11 having upwardly projecting walls is formed on the interior side of the femoral component 10 and includes an interior region that intersects the opening 12. The extension 24 includes a posterior surface 25 that is intended to be in frictional contact with the posterior surface 14 of the opening 12 when the joint is flexed. The resistance generated when the extension 24 bears against the posterior surface 14 of the opening 12 in the femoral component 10 is intended to simulate the resistance that would have been generated by a healthy posterior cruciate ligament (PCL).

Cam and post mechanisms have been manufactured that partially replace the function of an ACL by creating a cam surface between the anterior surface of the extension 24 and the anterior surface of the opening 12; however, this solution provides only a partial substitution of an ACL because the anterior side of the extension 24 is at best able to contact the anterior side of the opening only between 0 to 20 degrees of flexion.

The lack of an anatomically correct replacement may result in a TKR having reduced functionality as compared to the original knee. This may create difficulties during physical therapy following surgery, as well as limit the patient's ability or desire to participate in physical activity following therapy. Virtually all modern total knee replacements sacrifice the ACL or inadequately substitute it with a crude cam and post mechanism, thus leaving the reconstructed knee with kinematics similar to that of an ACL-deficient knee. Normal knee kinematics therefore remain elusive. In addition, the lack of proper interplay between an ACL and PCL (which together drive normal knee kinematics) leaves the TKR reconstruction short of producing a relatively normal knee for the patient.

Given the complexity of the mechanics of a knee joint and the difficulty for patients to adjust to an artificial knee after surgery, an anatomically correct knee replacement system is needed that more accurately simulates the resilience and support formerly provided by the removed ligaments. In order to provide a more anatomically correct TKR, prosthesis embodiments that replicate the function provided by both the ACL and PCL are desirable.

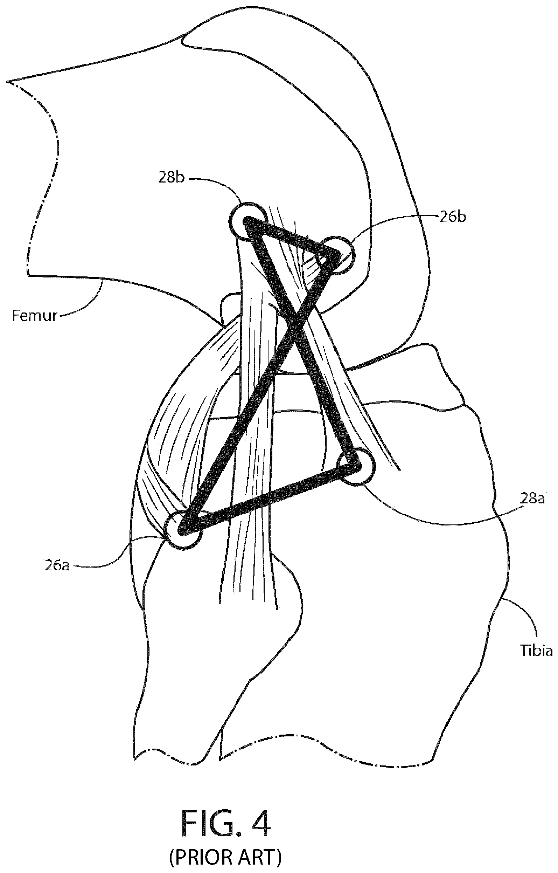

Referring now to FIG. 4, a healthy human knee is illustrated with a loop 30, representing an exemplary artificial ACL/PCL ligament, drawn over the location of the original anatomical ACL and PCL. The section of the loop 30 constituting the artificial PCL is bounded by points 26A and 26B. The section of the loop 30 constituting the artificial ACL is bounded by points 28A and 28B.

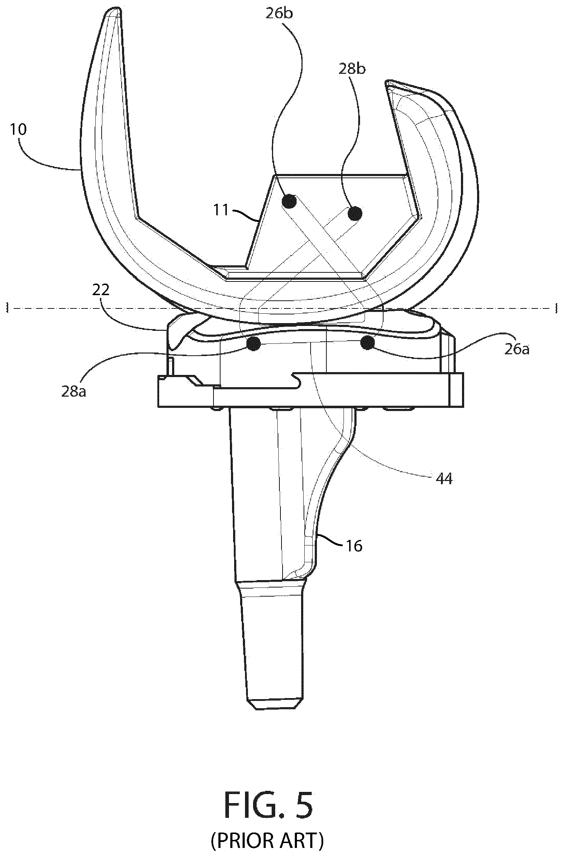

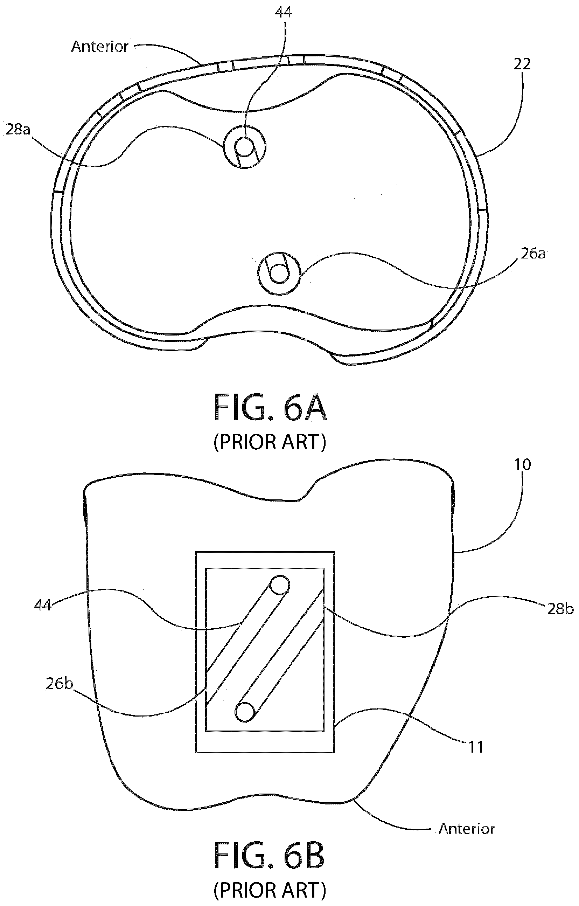

Referring now to FIG. 5, 6A, and 6B illustrating a knee prosthesis disclosed in U.S. Patent App. Pub. No. 2017/0252173 to Garino, the connection points 26a, 26b, 28a, and 28b of the artificial material, provided as ligament 44, and the lengths spanning between the connection points, provided as an outline of the artificial ligament 44, are configured to simulate the dimensions and attachment points of the ACL and PCL in a human knee, as illustrated in FIG. 4. At least one length of artificial ligament may be provided to connect the articular insert 22 and femoral component 10 of a TKR.

While U.S. Patent App. Pub. No. 2017/0252173 to Garino provides solutions to these complexities, developments in this area are continually sought in the interest of improving the mechanics of a knee joint.

Both US 2022/354657 A1 and EP 0103697 A1 respectively disclose an artificial knee joint prosthesis in which a femoral component and a tibial component are mechanically coupled by an interlocking gear connection comprising a plurality of pins.

### SUMMARY OF THE INVENTION

According to an embodiment of the present invention, a knee joint prosthesis is configured to move between an extended position and a flexion position. The prosthesis includes a femoral component configured to be mounted to a femur, the femoral component having a femoral gear including a plurality of conical shaped posts arranged along a helical trajectory. The prosthesis also includes a tibial component configured to (i) be mounted either directly or indirectly to a tibia, and (ii) engage the femoral component. The tibial component has a tibial gear including a plurality of recesses that are configured to mesh with the conical shaped posts of the femoral gear. The tibial gear has two intersecting portions including a horizontally-oriented curved portion and a vertical portion, wherein each portion includes at least one of the recesses. The plurality of conical shaped posts follow a helical trajectory to cause movement of the tibial component in either a medial or lateral direction as the knee joint prosthesis is moved to the extended position.

### BRIEF DESCRIPTION OF THE FIGURES

FIG. 1A is a side view of a femoral component for a knee joint prosthesis known by those of ordinary skill in the art.
FIG. 1B is a bottom view of the femoral component of FIG. 1A.
FIG. 2A is a top view of a tibial component for a knee joint prosthesis known by those of ordinary skill in the art.
FIG. 2B is a side view of the tibial component of FIG. 2A.
FIG. 3A is a top view of an articular insert for a knee joint prosthesis known by those of ordinary skill in the art.
FIG. 3B is a front view of the articular insert of FIG. 3B mounted on the baseplate of a tibial component.
FIG. 4 is a side view of a knee joint illustrating the anatomical location of the ACL and PCL and the configuration of an artificial material intended to replace the ACL and PCL.
FIG. 5 is a side view of a femoral component, articular insert, and artificial ligament for a TKR according to the prior art.
FIG. 6A is a top view of a cross-section along line I-I of FIG. 5.
FIG. 6B is a bottom view of a cross-section along line I-I of FIG. 5.
FIG. 7A is an assembled view of a modular knee joint prosthesis as viewed from the top, left and posterior sides, and according to the prior art.
FIG. 7B is an exploded view of the modular knee joint prosthesis of FIG. 7A.
FIGs. 7C-7F depict side elevation, top plan, rear/posterior elevation and front/anterior elevation views, respectively, of the modular knee joint prosthesis of FIG. 7A.
FIG. 7G depicts a cross-sectional view of the modular knee joint prosthesis of FIG. 7F taken along the lines 7G-7G, wherein the modular knee joint prosthesis is shown in an extended position.
FIG. 7H depicts another cross-sectional view of the modular knee joint prosthesis like FIG. 7G, but with the modular knee joint prosthesis shown in a flexion position.
FIG. 8A is an assembled view of a modular knee joint prosthesis according to an embodiment of the invention.
FIG. 8B is an exploded view of the modular knee joint prosthesis of FIG. 8A.
FIGs. 8C-8F depict side elevation, top plan, rear elevation and front elevation views, respectively, of the modular knee joint prosthesis of FIG. 8A.
FIG. 8G depicts a cross-sectional view of the modular knee joint prosthesis of FIG. 8F taken along the lines 8G-8G, wherein the modular knee joint prosthesis is shown in an extended position.
FIG. 8H depicts another cross-sectional view of the modular knee joint prosthesis like FIG. 8G, but with the modular knee joint prosthesis shown in a flexion position.
FIG. 8I depicts an exploded view of the modular knee joint prosthesis of FIG. 8A in addition to a femoral bone, a tibial bone and a tibial baseplate.

### DETAILED DESCRIPTION OF THE PREFERRED EMBODIMENTS

The present invention provides various embodiments of a knee joint prosthesis. In the figures, 'A' represents the anterior side or direction, 'P' represents the posterior side or direction, 'M' represents the medial side or direction, and 'L' represents the lateral side or direction.

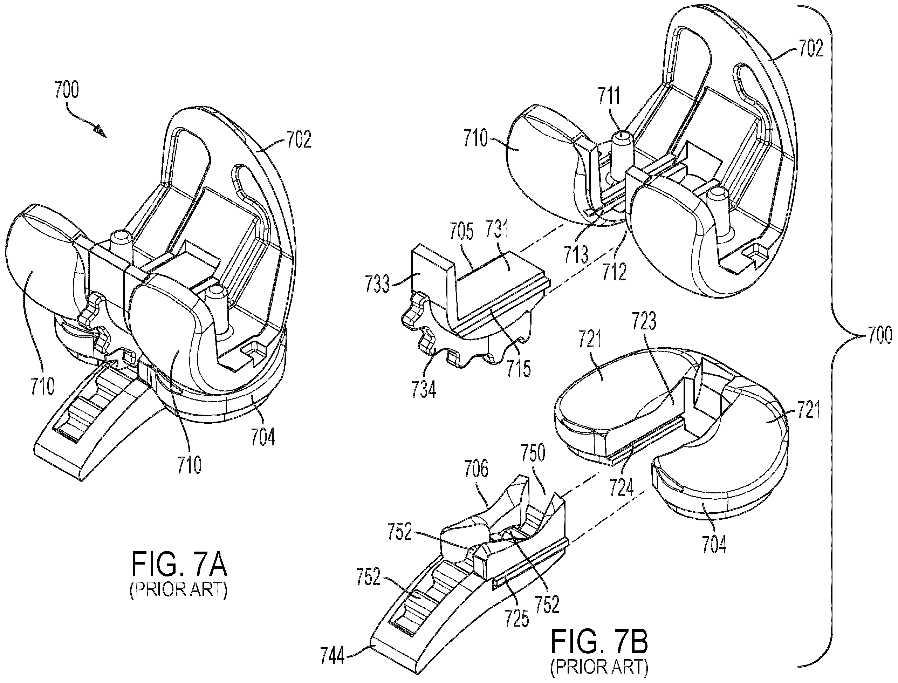

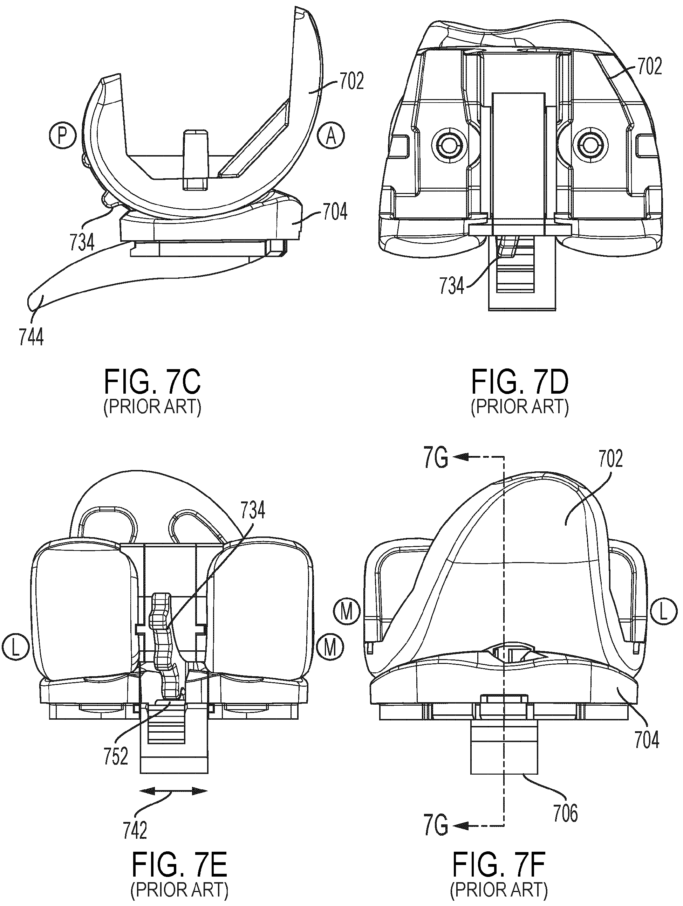

As is described in U.S. Patent No. 11,419,731 to Garino, FIGs. 7A-7H depict a modular knee joint prosthesis 700 according to the prior art. Although a prosthesis 700 for a left knee is shown and described hereinafter it should be understood that the right knee prosthesis is substantially similar and the explanation that follows also applies to the right knee prosthesis.

Prosthesis 700 generally comprises a modular femoral component 702, a femoral insert 705 that is configured to be mounted to the femoral component 702, a modular articular component 704, and a tibial insert 706 that is configured to be mounted to the articular component 704.

Modular femoral component 702 includes a U-shaped body having opposing condyles 710. Pins 711 extend upwardly from the inside surface of component 702 for implantation of femoral component 702 into a femur bone, as is known in the art. A rectangular cutout 712 is defined in the femoral component 702 at a central location between the condyles 710. Cutout 712 extends in the posterior-anterior direction and in the sagittal plane. Cutout 712 includes an opening at the posterior side of component 702. Cutout 712 extends through the entire wall thickness of component 702. Cutout 712 includes three interconnected and interior facing sides, namely, a lateral side extending in the sagittal plane, a medial side extending in the sagittal plane, and an anterior side that connects the lateral and medial sides. The posterior side of cutout 712 is open for receiving the insert 705.

A relief is formed on each of the opposing medial and lateral sides of cutout 712. Together the reliefs form a slot or track 713 that is configured to receive rails 715 (i.e., shoulders) formed on opposing sides of femoral insert 705 such that insert 705 can be installed on component 702. It should be understood that track 713 may be disposed on insert 705 and rails 715 may be disposed on component 702 to achieve either the same or a similar result.

It should be understood that means for mounting insert 705 to component 702, other than the tracks 713 and rails 715, may be provided. The means for mounting may alternatively comprise a slot, guide, rail, snap feature, friction fit, interference fit, fastener (screw, bolt, nut), weld, adhesive, dovetail (or other mating surface).

Modular articular component 704 is configured to reside on a top mounting portion of a tibial component (not shown) for interfacing with femoral component 702. Articular component 704 is configured to be mounted to the tibial baseplate 16 (see FIGs. 2B and 5) that is fixedly mounted to the tibia. Alternatively, and although not shown, articular component 704 may be integrated with the tibial baseplate 16 to form a single unitary tibial component that is fixedly mounted to the tibia. Thus, a "tibial component" may comprise either articular component 704 (alone) or both articular component 704 and tibial baseplate 16.

Articular component 704 includes two concave surfaces 721 for physically engaging with the convexly shaped condyles 710 of femoral component 702 as prosthesis 700 is moved between the flexion and extension positions. A rectangular cutout 723 is defined in the articular component 704 at a central location between the concave surfaces 721. Cutout 723 extends in the posterior-anterior direction and in the sagittal plane. Cutout 723 includes an opening at the posterior side of articular component 704. Cutout 723 extends through the wall thickness of articular component 704. Cutout 723 includes three interconnected and interior facing sides, namely, a lateral side extending in the sagittal plane, a medial side extending in the sagittal plane, and an anterior side that connects the lateral and medial sides. A relief is formed on each of the opposing medial and lateral sides of cutout 723. Together the reliefs form a slot or track 724 that is configured to receive rails 725 (shoulders) formed on opposing sides of tibial insert 706 such that insert 706 can be installed on articular component 704 by sliding rails 725 along tracks 724. It should be understood that track 724 may be disposed on insert 706 while rails 725 may be disposed on articular component 704 to achieve either the same or a similar result.

It should be understood that means for mounting insert 706 to articular component 704, other than the tracks 724 and rails 725, may be provided. The means for mounting may alternatively comprise a slot, guide, rail, snap feature, friction fit, interference fit, fastener (screw, bolt, nut), weld, adhesive, dovetail (or other mating surface).

Femoral component 702 and articular component 704 are modular components that are common amongst the previously described prior art examples. Femoral component 702 and articular component 704 may together form a modular sub-assembly.

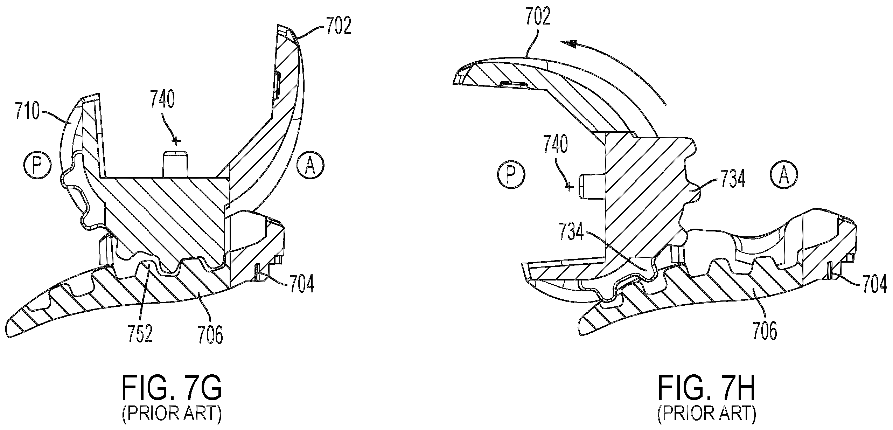

Turning now to the components that are capable of being selectively installed on those modular components 702 and 704, femoral insert 705 is configured to be mounted to femoral component 702, and tibial insert 706 is configured to be mounted to articular component 704 (otherwise referred to tibial component 704). Femoral and tibial inserts 705 and 706 work together to guide motion of the prosthesis 700 between the flexion and extension positions shown in FIGs. 7G and 7H.

Femoral insert 705 comprises an L-shaped body. The body defines an elongated horizontal portion 731 on which rails 715 protrude from the opposing side surfaces thereof. The horizontal portion 731 is sized to fit snugly within cutout 712 of femoral component 702. A vertical portion 733 of the body extends orthogonally from the horizontal portion 731. In an assembled form, the vertical portion 733 sits flush with and bridges the condyles 710 of femoral component 702. Vertical portion 733 also serves as a finger tab for ease of manual insertion and/or removal of the insert 705 onto/from the femoral component 702. Femoral insert 705 may be either releasably or non-releasably connected to femoral component 702.

A gear 734, in the form of a convex surface, protrudes outwardly from the front and rear exterior facing surfaces of the L-shaped body of insert 705. Gear 734 comprises a plurality of individual gear teeth having rounded outer surfaces. The gear teeth are uniformly spaced apart along the outer perimeter of gear 734. Gear 734 follows a helical path, as can be viewed in FIGs. 7D and 7E. Specifically, gear 734 curves about (i) its own axis 740 (FIGs. 7G and 7H) extending in the medial-lateral direction and (ii) an axis that extends in the anterior-posterior direction (see FIG. 7E). Stated differently, gear 734 curves along two different axes that are oriented orthogonal to each other. Gear 734 resides on the front and rear surfaces of the L-shaped body, whereas the rails 715 are disposed on the left and right sides of the body, and the top and rear facing surfaces are planar. Femoral insert 705 may be a unitary, monolithic component.

Tibial insert 706 comprises an elongated rectangular body. Rails 725 extend in the anterior-posterior direction and protrude from the opposing side surfaces of the body. Rails 725 are sized to fit within the track 724 formed on articular component 704, as described above, such that rails 725 slide in their corresponding tracks 724. A finger tab 744 is defined on the lower posterior edge of insert 706 to ease manual handling of insert 706. Tibial insert 706 may be either releasably or non-releasably connected to articular component 704.

A curved channel 750 extends in the anterior-posterior direction through the body of the insert 706. The channel 750 has opposing curved sidewalls that face each other. The channel 750 curves about a vertical axis (in the superior-inferior direction or along the sagittal plane), as shown in FIG. 7B, for example. The base surface of the channel 750 has a series of gear teeth 752 extending upwards therefrom for meshing with the teeth of gear 734. The base surface may be flat (with the exception of the teeth 752), or the base surface may be curved about axis 740 (FIG. 7H). Insert 706 may be a unitary, monolithic component.

Turning now to FIGs. 7G and 7H, the gear teeth 752 on the articular component 704 are meshed with the gear teeth 734 of the femoral component 702, such that the articular component 704 rotates on the femoral component 702, or vice versa. As the femoral component 702 rotates in a posterior direction to the flexion position shown in FIG. 7H (as designated by the arrow), the meshing teeth do not slide past one another. The slight rotation of the articular component 704 in the medial-lateral direction as the prosthesis 700 is moved between the flexion and extension positions, as a result of the curvature of gear teeth 734 in the medial lateral direction, mimics the slight rotation experienced in a real knee joint.

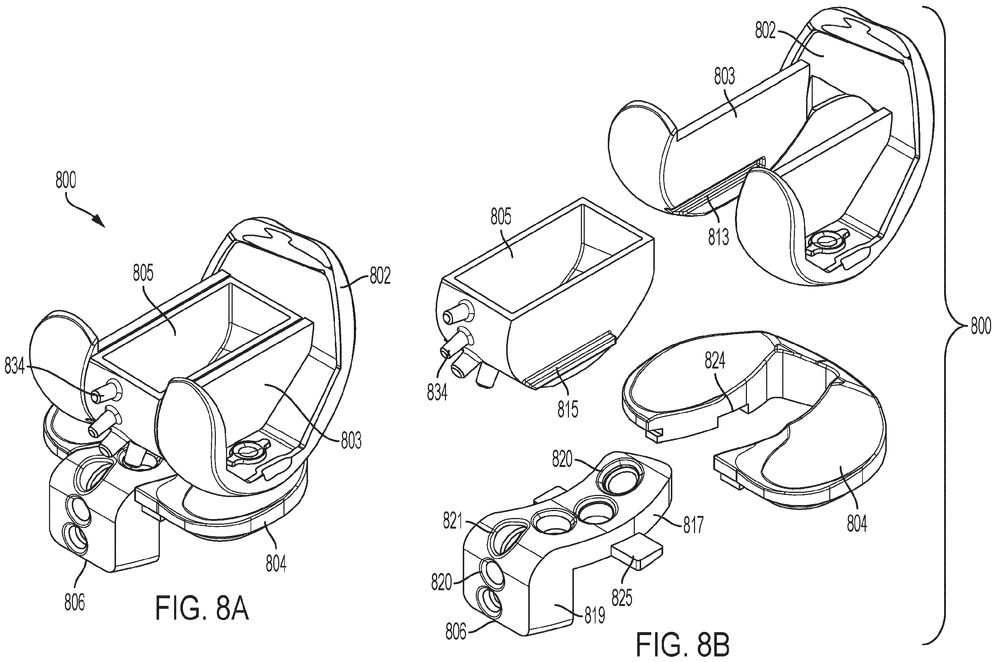

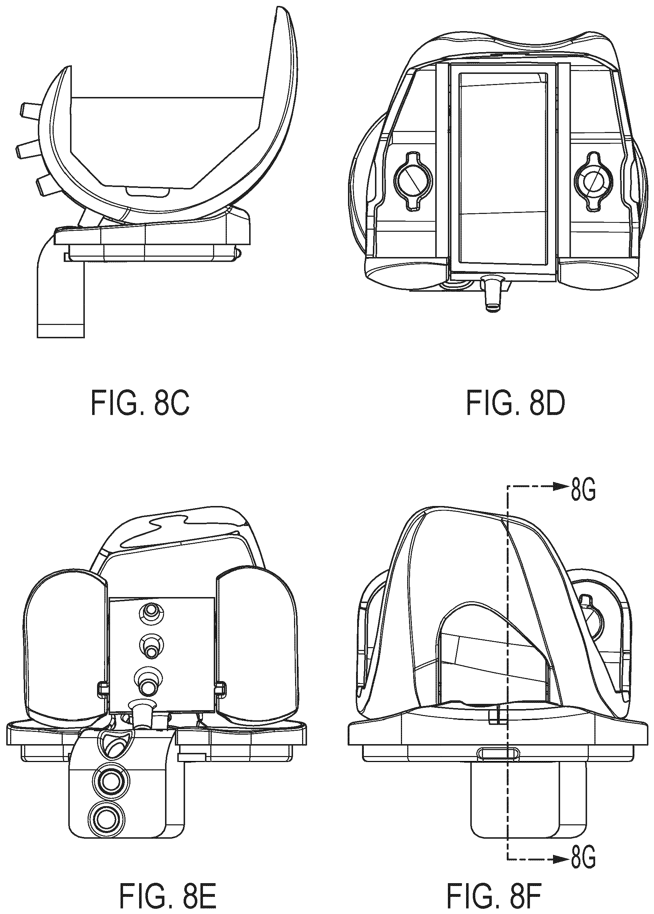

FIGs. 8A-8H depict a modular knee joint prosthesis 800 according to an embodiment of the invention.

It should be understood that prosthesis 800 is similar to prosthesis 700 and only the primary differences therebetween will be described hereinafter. Thus, the details provided above with respect to prosthesis 700 also apply to prosthesis 800, unless explained otherwise hereinafter.

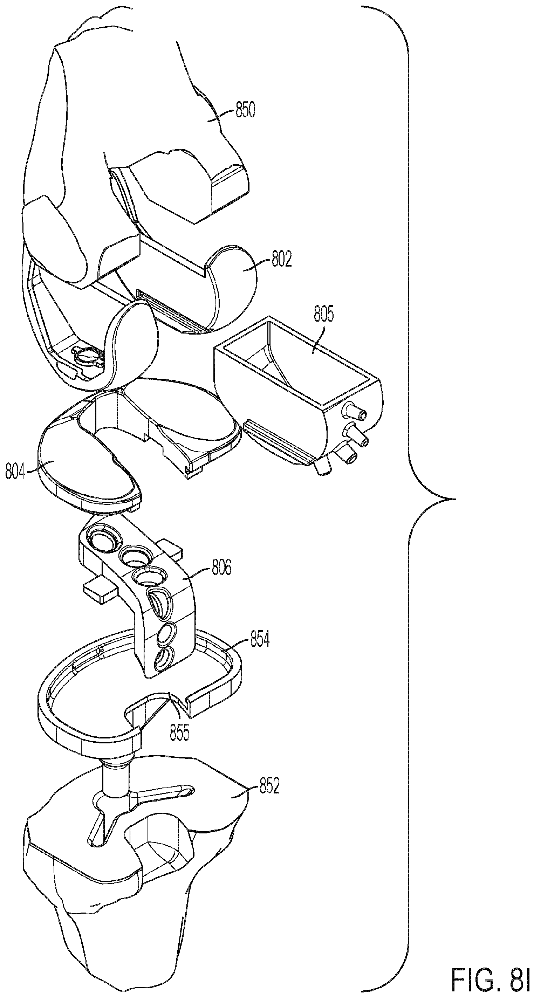

Prosthesis 800 includes femoral component 802, articular component 804, femoral insert 805 (also referred to herein as a femoral gear), and a tibial insert 806 (also referred to herein as a tibial gear). FIG. 8I depicts an exploded view of the prosthesis 800 in addition to a femoral bone 850, a tibial bone 852 and a tibial baseplate 854 (like baseplate 16) for use with prosthesis 800. Tibial baseplate 854 may also be considered as forming part of prosthesis 800.

Femoral component 802 is similar to femoral component 702 with the exception that it includes vertical side walls 803 extending above the tracks 813. Articular component 804 is also similar to articular component 704 with the exception that it includes a rectangular recess 824 on the bottom surface in lieu of a track (like track 724). In assembled form, opposing tabs 825 on tibial insert 806, which extend in the lateral-medial direction, are inserted into recess 824, thereby captivating articular component 804 to tibial insert 806.

Turning now to femoral insert 805, insert 805 includes a hollow half-cylinder shaped body. Rails 815 extending in the posterior-anterior direction are disposed on opposing planar side walls of the body for engaging in tracks 813 of femoral component 802. In an assembled form, the planar side walls of the body are both supported and constrained in the medial-lateral direction by the side walls 803 of femoral component 802, as shown in FIG. 8A. A series of gear teeth in the form of pegs 834 are disposed on the rounded posterior face of insert 805. Each peg 834 has a frusto-conical shape, as shown. Alternatively, each peg 834 could have cylindrical, triangular, conical, rectangular or trapezoidal shape (as viewed in cross-section, for example). The free end surface of such a cylindrically shaped peg could be flat or rounded. However, it was discovered that the frusto-conical shaped pegs 834 performed particularly well during testing in the way of smooth movement. Pegs 834 may be arranged along a helical path, as shown in FIG. 8E, to mimic motion of the knee (as was described above with reference to prosthesis 700). The helical path is curved to drive rotation around the medial condyle forcing more translation of the lateral condyle as the knee is flexed. Alternatively, pegs 834 may be arranged along a straight path.

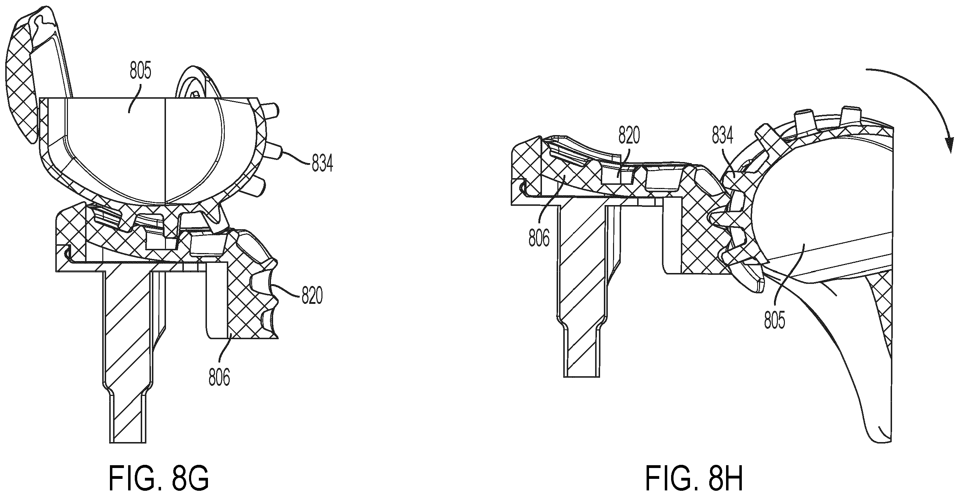

Pegs 834 may be evenly spaced apart in a uniform manner, or unevenly spaced apart. For example, to enhance stability of the prosthesis, adjacent pegs 834 that are active (i.e., meshed) in the deep flexion position (FIG. 8H) of prosthesis 800 may be positioned closer together than the adjacent pegs 834 that are active in the extension position (FIG. 8G) of prosthesis 800. Also, the protruding length, thickness, width, etc. of each peg 834 may be either uniform or non-uniform. Specifically, adjacent pegs 834 that are active (i.e., meshed) in the deep flexion position (FIG. 8H) of prosthesis 800 may have a different protruding length, thickness, width, etc. than the adjacent pegs 834 that are active in the extension position (FIG. 8G) of prosthesis 800.

Turning now to tibial insert 806, insert 806 includes a substantially L-shaped body including a horizontally-oriented curved portion 817 and a substantially vertical portion 819. The curvature of the horizontally-oriented curved portion 817 may match the curvature of the curved surface of femoral insert 805. The vertical portion 819 extends downwardly. The vertical portion 819 is provided such that the femoral insert can engage the tibial insert at a position that is far posterior and even slightly down the back of the tibia in the deep flexion state shown in FIG. 8H.

In an assembled state, the vertical portion 819 passes through a rectangular channel 855 (FIG. 8I) formed in tibial baseplate 854. A series of concave recesses 820 are formed on the exterior surface of insert 806. The outer perimeter of each recess 820 is substantially circular with the exception of recess 821, which has a teardrop shaped outer perimeter. The perimeter shape of recess 821 may also be described as elliptical. Recess 821 is formed at the intersection of portions 817 and 819. Recesses 820/821 may be arranged along a helical path, as shown in FIGs. 8B and 8E, to mimic motion of the knee (as was described above). Alternatively, recesses 820/821 may be arranged along a straight path. Although the circular/elliptical shape of recesses hase been found to yield smooth kinematics for the prosthesis 800, it should be understood that the outer perimeter of recesses 820/821 can vary and may take a rectangular, triangular or trapezoidal shape, for example.

It should be understood that the arrangement of the pegs and recesses may be reversed. In other words, the pegs 834 may be disposed on the tibial insert 806 and the recesses 820 may be disposed on the femoral insert 805.

Prosthesis 800 includes access openings in the tibial and femoral components like those openings represented by items 712 and 723, which enable a surgeon to access an intramedullary rod in the case of fracture.

Although the femoral insert 805 is shown as a separate component, it may be formed as a unitary component along with the femoral component 802. Similarly, although the tibial insert 806 is shown as a separate component, it may be formed as a unitary component along with either the articular component 804 or the tibial baseplate 854.

The geometries of the femoral inserts and tibial inserts can vary. A plurality of different femoral inserts and tibial inserts can be provided with one femoral component 802 and one articular component 804 as a kit, if so desired. In use, different femoral and tibial inserts may be selected by the surgeon based upon various factors including age, gender, ailments, and size of the patient, for example.

The components of the knee joint prosthesis may be made of the same or similar material. In general, however, all materials are preferably inert, not prone to cause infection, and otherwise safe and approved for use as a surgical implant. Exemplary materials include polyethylene, surgically approved metal alloys, surgically approved ceramic materials, or a combination thereof. Any well-known materials in the field of surgical implants may be used to fabricate any of the various embodiments or portions thereof according to the present invention.

While preferred embodiments of the invention have been shown and described herein, it will be understood that such embodiments are provided by way of example only. Numerous variations, changes, and substitutions may occur to those skilled in the art without departing from the scope of the invention. Accordingly, it is intended that the appended claims cover all such variations that fall within the scope of the invention, which is solely defined by said claims.

## Claims

1. A knee joint prosthesis (800) configured to move between an extended position and a flexion position, the knee joint prosthesis comprising:
a femoral component (802) configured to be mounted to a femur, the femoral component having a femoral gear (805) including a plurality of posts (834) arranged along a helical trajectory; and
a tibial component (804) configured to (i) be mounted either directly or indirectly to a tibia, and (ii) engage the femoral component, the tibial component having a tibial gear (806) including a plurality of recesses (820, 821) that are configured to mesh with the posts of the femoral gear,
wherein the plurality of posts follow a helical trajectory to cause movement of the tibial component in either a medial or lateral direction as the knee joint prosthesis is moved to the extended position,
**characterized in that** the tibial gear has two intersecting portions including a horizontally-oriented curved portion (817) and a vertical portion (819), wherein each portion includes at least one of the recesses.

2. The knee joint prosthesis of claim 1, wherein the femoral and tibial gears are configured to be meshed together in both the extended and flexion positions.

3. The knee joint prosthesis of claim 1, wherein the plurality of recesses also follow a helical trajectory to cause movement of the tibial component in either the medial or lateral direction as the knee joint prosthesis is moved to the extended position.

4. The knee joint prosthesis of claim 1, wherein the knee joint prosthesis is a modular knee joint prosthesis, wherein the femoral gear is a femoral insert (805) that is removably mounted in a first cutout or opening that is defined in a central region of the femoral component, and wherein the tibial gear is a tibial insert (806) that is removably mounted in a second cutout or opening that is defined in a central region of the tibial component.

5. The knee joint prosthesis of claim 4, wherein the femoral insert is removably mounted within the first cutout or opening of the femoral component by a rail and slot engagement.

6. A kit comprising the knee joint prosthesis of claim 4, a plurality of different femoral gears and a plurality of different tibial gears.

7. The knee joint prosthesis of claim 1, wherein the femoral gear and the femoral component are formed as one unitary component.

8. The knee joint prosthesis of claim 1, wherein the tibial gear and the tibial component are formed as one unitary component.

9. The knee joint prosthesis of claim 1, wherein the tibial component is an articular component having concave bearing surfaces shaped to engage condyles on the femoral component.

10. The knee joint prosthesis of claim 1, wherein the tibial component is a tibial baseplate that is configured to be directly mounted to a tibial bone.

11. The knee joint prosthesis of claim 1, wherein the femoral component comprises opposing condyles, and the femoral and tibial gears are positioned in the medial-lateral direction at a location between the opposing condyles.

12. The knee joint prosthesis of claim 1, wherein the posts each have a conical or frusto-conical shape.

13. The knee joint prosthesis of claim 1, wherein the posts have different shapes and the recesses have different shapes.

14. The knee joint prosthesis of claim 1, wherein the posts are non-uniformly spaced apart.

## Patentansprüche

1. Eine Kniegelenkprothese (800), die so ausgelegt ist, dass sie sich zwischen einer Streckposition und einer Beugungsposition bewegen kann, wobei die Kniegelenkprothese umfasst:
eine Femurkomponente (802), die so ausgelegt ist, dass sie an einem Femur befestigt werden kann, wobei die Femurkomponente ein Femurzahnrad (805) aufweist, das eine Vielzahl von Zapfen (834) umfasst, die entlang einer spiralförmigen Bahn angeordnet sind; und
eine Tibiakomponente (804), die so ausgelegt ist, dass sie (i) entweder direkt oder indirekt an einer Tibia befestigt wird und (ii) mit der Femurkomponente in Eingriff kommt, wobei die Tibiakomponente ein Tibiazahnrad (806) aufweist, das eine Vielzahl von Aussparungen (820, 821) umfasst, die so ausgelegt sind, dass sie mit den Zapfen des Femurzahnrads in Eingriff kommen,
wobei die mehreren Zapfen einer spiralförmigen Bahn folgen, um eine Bewegung der Tibiakomponente entweder in medialer oder lateraler Richtung zu bewirken, wenn die Kniegelenkprothese in die gestreckte Position bewegt wird, **dadurch gekennzeichnet, dass** das Tibiagewinde zwei sich schneidende Abschnitte aufweist, die einen horizontal ausgerichteten, gekrümmten Abschnitt (817) und einen vertikalen Abschnitt (819) umfassen, wobei jeder Abschnitt mindestens eine der Aussparungen aufweist.

2. Kniegelenkprothese nach Anspruch 1, wobei das Femur- und das Tibiazahnrad so konfiguriert sind, dass sie sowohl in der gestreckten als auch in der gebeugten Position miteinander in Eingriff stehen.

3. Kniegelenkprothese nach Anspruch 1, wobei die mehreren Aussparungen ebenfalls einer spiralförmigen Bahn folgen, um eine Bewegung der Tibiakomponente entweder in medialer oder lateraler Richtung zu bewirken, wenn die Kniegelenkprothese in die gestreckte Position bewegt wird.

4. Kniegelenkprothese nach Anspruch 1, wobei es sich bei der Kniegelenkprothese um eine modulare Kniegelenkprothese handelt, wobei das Femurgetriebe ein Femureinsatz (805) ist, der entnehmbar in einer ersten Aussparung oder Öffnung angebracht ist, die in einem zentralen Bereich der Femurkomponente definiert ist, und wobei das Tibia-Getriebe ein Tibia-Einsatz (806) ist, das entfernbar in einer zweiten Aussparung oder Öffnung angebracht ist, die in einem zentralen Bereich der Tibiakomponente definiert ist.

5. Kniegelenkprothese nach Anspruch 4, wobei der Femureinsatz durch einen Schienen- und Schlitz-Eingriff entfernbar in der ersten Aussparung oder Öffnung der Femurkomponente angebracht ist.

6. Ein Bausatz, umfassend die Kniegelenkprothese nach Anspruch 4, eine Vielzahl verschiedener Femur-Zahnräder und eine Vielzahl verschiedener Tibia-Zahnräder.

7. Kniegelenkprothese nach Anspruch 1, wobei das Femur-Zahnrad und die Femurkomponente als ein einteiliges Bauteil ausgebildet sind.

8. Kniegelenkprothese nach Anspruch 1, wobei das Tibia-Zahnrad und die Tibia-Komponente als ein einteiliges Bauteil ausgebildet sind.

9. Kniegelenkprothese nach Anspruch 1, wobei die Tibia-Komponente eine Gelenkkomponente mit konkaven Lagerflächen ist, die so geformt sind, dass sie mit den Kondylen an der Femurkomponente in Eingriff kommen.

10. Kniegelenkprothese nach Anspruch 1, wobei die Tibiakomponente eine Tibia-Grundplatte ist, die so konfiguriert ist, dass sie direkt an einem Tibiaknochen befestigt werden kann.

11. Kniegelenkprothese nach Anspruch 1, wobei die Femurkomponente gegenüberliegende Kondylen umfasst und das Femur- und das Tibiazahnrad in mediolateraler Richtung an einer Stelle zwischen den gegenüberliegenden Kondylen positioniert sind.

12. Kniegelenkprothese nach Anspruch 1, wobei die Zapfen jeweils eine konische oder kegelstumpfförmige Gestalt aufweisen.

13. Kniegelenkprothese nach Anspruch 1, wobei die Zapfen unterschiedliche Gestalten und die Aussparungen unterschiedliche Gestalten aufweisen.

14. Kniegelenkprothese nach Anspruch 1, wobei die Zapfen in ungleichmäßigen Abständen voneinander angeordnet sind.

## Revendications

1. Prothèse de l'articulation du genou (800) conçue pour se déplacer entre une position d'extension et une position de flexion, ladite prothèse comprenant :
un composant fémoral (802) conçu pour être fixé à un fémur, ledit composant fémoral comportant un engrenage fémoral (805) comprenant une pluralité de tiges (834) disposées selon une trajectoire hélicoïdale ; et
un composant tibial (804) conçu pour (i) être fixé, directement ou indirectement, à un tibia, et (ii) s'engager avec le composant fémoral, le composant tibial comportant un engrenage tibial (806) comprenant une pluralité d'évidements (820, 821) qui sont conçus pour s'engrener avec les tiges de l'engrenage fémoral,
dans laquelle la pluralité de tenons suit une trajectoire hélicoïdale pour provoquer un déplacement du composant tibial soit dans une direction médiale, soit dans une direction latérale, lorsque la prothèse de l'articulation du genou est amenée en position d'extension, **caractérisée en ce que** l'engrenage tibial comporte deux parties qui se croisent, comprenant une partie courbe orientée horizontalement (817) et une partie verticale (819), chaque partie comportant au moins l'un des évidements.

2. Prothèse de l'articulation du genou selon la revendication 1, dans laquelle les engrenages fémoral et tibial sont configurés pour s'engrener l'un avec l'autre aussi bien en position d'extension qu'en position de flexion.

3. Prothèse de l'articulation du genou selon la revendication 1, dans laquelle la pluralité d'évidements suit également une trajectoire hélicoïdale pour provoquer un déplacement du composant tibial soit dans la direction médiale, soit dans la direction latérale lorsque la prothèse de l'articulation du genou est amenée en position d'extension.

4. Prothèse de genou selon la revendication 1, dans laquelle la prothèse de genou est une prothèse de genou modulaire, dans laquelle l'engrenage fémoral est un insert fémoral (805) monté de manière amovible dans une première découpe ou ouverture définie dans une région centrale du composant fémoral, et dans laquelle l'engrenage tibial est un insert tibial (806) qui est monté de manière amovible dans une deuxième découpe ou ouverture définie dans une région centrale du composant tibial.

5. Prothèse de genou selon la revendication 4, dans laquelle l'insert fémoral est monté de manière amovible à l'intérieur de la première découpe ou ouverture du composant fémoral par un engagement rail-fente.

6. Kit comprenant la prothèse de l'articulation du genou selon la revendication 4, une pluralité d'engrenages fémoraux différents et une pluralité d'engrenages tibiaux différents.

7. Prothèse de l'articulation du genou selon la revendication 1, dans laquelle l'engrenage fémoral et le composant fémoral sont formés en un seul composant unitaire.

8. La prothèse de l'articulation du genou selon la revendication 1, dans laquelle l'engrenage tibial et le composant tibial sont formés en un seul composant unitaire.

9. La prothèse de l'articulation du genou selon la revendication 1, dans laquelle le composant tibial est un composant articulaire présentant des surfaces d'appui concaves formées pour s'engager avec les condyles du composant fémoral.

10. Prothèse de l'articulation du genou selon la revendication 1, dans laquelle le composant tibial est une plaque de base tibiale configurée pour être montée directement sur l'os tibial.

11. Prothèse de l'articulation du genou selon la revendication 1, dans laquelle le composant fémoral comprend des condyles opposés, et les engrenages fémoral et tibial sont positionnés dans la direction médial-latéral à un emplacement situé entre les condyles opposés.

12. Prothèse de l'articulation du genou selon la revendication 1, dans laquelle les tiges présentent chacune une forme conique ou tronconique.

13. Prothèse de l'articulation du genou selon la revendication 1, dans laquelle les tiges présentent des formes différentes et les évidements présentent des formes différentes.

14. Prothèse de l'articulation du genou selon la revendication 1, dans laquelle les tiges sont espacées de manière non uniforme.
